# EUROPEAN PATENT SPECIFICATION

(11) **EP 2 788 323 B1**
(45) Date of publication and mention of the grant of the patent: **11.01.2017**
(21) Application number: 12773344.2
(22) Date of filing: 22.10.2012
(51) Int. Cl.: C07D 207/16

(54) **NEW PROCESS AND INTERMEDIATES FOR THE SYNTHESIS OF VILDAGLIPTIN**
NEUES VERFAHREN UND ZWISCHENPRODUKTE ZUR SYNTHESE VON VILDAGLIPTIN
NOUVEAU PROCÉDÉ ET NOUVEAUX INTERMÉDIAIRES POUR LA SYNTHÈSE DE VILDAGLIPTINE

(30) Priority: 06.12.2011 IT MI20112224
(43) Date of publication of application: 15.10.2014
(73) Proprietor: Chemelectiva Srl., 28100 Novara (IT)
(72) Inventor: CASTALDI, Graziano, I-28100 Novara (NO) (IT); OLDANI, Erminio,, I-28100 Novara (NO) (IT); BARATELLA, Marco, I-28100 Novara (NO) (IT)
(74) Representative: Longoni, Alessandra
(86) International application number: PCT/EP2012/070869
(87) International publication number: WO 2013/083326

(56) References cited:
- WO-A1-2004/092127
- WO-A2-2010/022690
- WO-A2-2011/012322
- EDWIN B VILLHAUER ET AL: "1-[[(3-Hydroxy-1-adamantyl)amino]acetyl]- 2-cyano-(S)-pyrrolidine: A Potent, Selective, and Orally Bioavailable Dipeptidyl Peptidase IV Inhibitor with Antihyperglycemic Properties", JOURNAL OF MEDICINAL CHEMISTRY, AMERICAN CHEMICAL SOCIETY, US, vol. 46, no. 13, 1 January 2003 (2003-01-01), pages 2774-2789, XP002663718, ISSN: 0022-2623, DOI: 10.1021/JM030091L [retrieved on 2003-05-24] cited in the application

## Description

The present invention relates to a new process for the preparation of vildagliptin and to intermediates for its synthesis.

Vildagliptin is an inhibitor of dipeptidyl-peptidase 4 (DPP-4), enzymes that degrade the incretin hormones, which is used in adults suffering from diabetes mellitus type 2 (non insulin-dependent diabetes) to improve the control of blood glucose levels. Incretins are hormones produced in the gastrointestinal region and they are mainly GLP-1 (Glucagon-Like Peptide 1) and GIP (Glucose-dependent Insulinotropic Peptide). They are secreted after meals, particularly GLP-1, and have the function of controlling glycemia in different ways: increase of insulin secretion by beta cells of the pancreas, decrease of glucagon secretion (insulin antagonist) by alpha cells of the pancreas, slowdown of motility and therefore gastric empty with the consequent decrease in appetite.

GLP-1 is rapidly degraded into an inactive peptide by DDP-4, moreover its production decreases when glycemia,decreases, its control over the latter is then calibrated and "when needed" thus avoiding ipersecretion of insulin and the consequent dangerous hypoglycemia.

In diabetic patients the natural action of GLP-1 is defective, it was therefore thought to restore this activity for exploiting it, particularly for the oral therapy of diabetes mellitus type 2, a disorder in which the pancreas is not able to produce enough insulin to control blood glucose levels or in which the body is not able to effectively use insulin with the consequent advantage of decreasing various and problematic adverse side effects due to an extended oral therapy with the traditional drugs.

Vildagliptin, acting as a DDP-4 inhibitor, inhibits the degradation of incretin hormones in the body, increasing their level in the blood and stimulating the pancreas to produce more insulin when there is a high glycemic level, thus decreasing the amount of glucose produced by the liver; it also decreases glucagone levels allowing the control of diabetes mellitus type 2. Vildagliptin can be used in combination with metformin, sulfonylureas or with a thiazolidinedione.

Vildagliptin is a compound of formula (I) 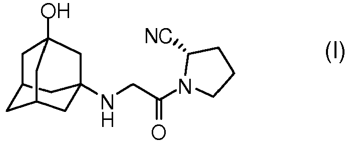 chemically known as (2S)-1-[2-[(3-hydroxy-1-adamantyl)amino]acetyl]pyrrolidin-2-carbonitrile disclosed in WO 00/34241.

Various vildagliptin syntheses are known in the literature.

WO 00/34241 (Novartis AG) discloses a process which provides for the reaction of a 2-cianopyrolidine derivative of formula (II) 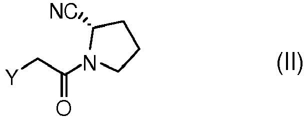 wherein Y is a reactive group such as chlorine, bromine or iodine; with 1-amino-3-adamantanol 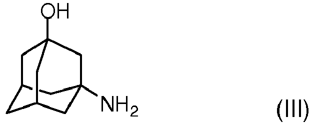 in the presence of a base or an inert solvent.

A similar synthesis is described in J. Med. Chem. 2003, 46, 2774-2789.

WO 04/92127 (Novartis AG) discloses a process which avoids the isolation of the 2-cianopyrrolidine derivative (II), a particularly irritating synthesis intermediate, which reacts directly with 1-amino-3-adamantanol (III) to give vildagliptin.

WO 08/84383 (Medichem SA) discloses a process which comprises the reaction between a 2-cianopyrrolidine derivative of formula (II) with 1-amino-3-adamantanol wherein the OH group is protected. The process gives vildagliptin with good yield and high optical purity and requires the final removal of the protecting group on the OH group.

WO 10/22690 (Zentiva KS) discloses a process for the preparation of vildagliptin which provides for the isolation of a 1-haloacetyl-2(S)-pyrrolidin-carboxamide of formula (IV) 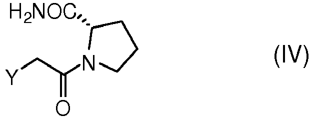 with a trialkylamine salt and its subsequent conversion into the corresponding ciano derivative of formula (II) which is reacted with 1-amino-3-adamantanol according to the conventional process for the synthesis of vildaglitin.

All known processes for the synthesis of vildagliptin are based on the reaction of a derivative of formula (II) with an optionally protected 1-amino-3-adamantanol (III). Furthermore the intermediate of formula (II) is generally obtained by the reaction between L-prolinamide and a halogenated compound, particularly chloroacetylchloride, a hard to handle, highly toxic, irritating, water and air sensitive liquid compound with a high reactivity which always requires the concomitant use of a base to neutralize the hydrochloric acid which develops during the reaction with the consequent formation of salts which complicate the purification and the isolation of the resultant intermediate. The reaction is then followed by the dehydration with trifluoroacetic anhydride, to obtain the corresponding acid, thus obtaining a process that requires an aqueous and difficult workup resulting in low yields. Finally, the processes disclosed in WO 00/34241 and WO 08/84383 involve the formation of the double N-alkylation product of formula (V) 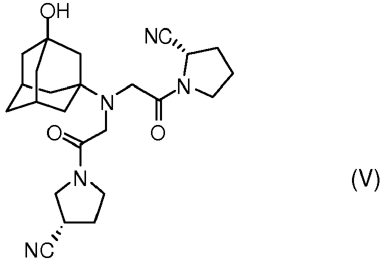 which causes difficulties in the purification step and reduction in yield and purity of the product.

We have now found a process for the preparation of vildagliptin characterized by a less difficult workup which leads to the formation of a reduced amount of double N-alkylation product thus obtaining the final product with high yields and high optical purity.

Therefore object of the present invention is a process for the synthesis of vildagliptin comprising the following steps:
a) the salification of L-prolinamide with haloacetic acid (XCH2COOH) in a suitable solvent to obtain the salt of formula (VI) 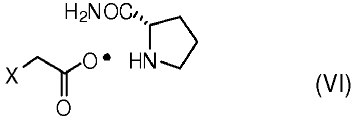 wherein X is bromine or chlorine.
b) the condensation of the salt of formula (VI) by reaction with a condensing agent and subsequent reaction with 1-amino-3-adamantanol in a suitable solvent to obtain the intermediate of formula (VII) without the isolation of the condensation product 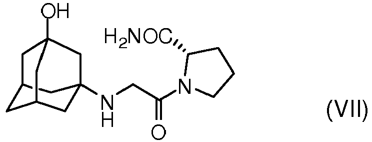
c) the dehydration of the compound (VII) to vildagliptin, by treatment with a dehydrating agent in a suitable solvent optionally in the presence of a base.

The process object of the present invention is characterized by a synthetic scheme based on new intermediates which allows to obtain vildagliptin with good yields, chemical purity >99.5% and optical purity >99.5% avoiding the drawbacks deriving from the use of 2-cyanopyrrolidine of formula (II) according to the prior art.

Furthermore the process object of the present invention leads to the formation of a reduced amount of double N-alkylation product.

Moreover, the chloroacetic acid used in the process is easier to handle compared to chloroacetylchloride, as it is a less toxic solid compound and it does not require the use of a base but of a condensing agent, thus avoiding the formation of salts which would make difficult the purification and isolation of the resultant intermediate.

The solvents used in steps a), b) and c), the same or different for each step, are preferably apolar solvents selected for example among tetrahydrofuran, methylene chloride, 2-methyltetrahydrofuran. Tetrahydrofuran or methylene chloride are preferred.

Preferably the reaction temperatures in steps a), b) and c), the same or different for each step, are from 0°C to 80°C, preferably from 20°C to 50°C.

Step a) of the process object of the present invention provides for the formation of the salt of formula (VI) by reaction between L-prolinamide and haloacetic acid (XCH₂COOH) wherein X can be chlorine or bromine, in a suitable solvent.

L-prolinamide and haloacetic acid are preferably used in stoichiometric amount.

At the end of step a) of the process object of the present invention, the compound of formula 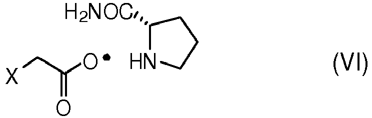 is obtained.

This is a new intermediate and is a further object of the present invention.

Step b) of the process object of the present invention provides for the condensation of the salt of formula (VI) by reaction with a condensing agent and subsequent reaction with 1-amino-3-adamantanol in a suitable solvent to obtain the intermediate of formula (VII) without the isolation of the condensation product.

The amount of 1-amino-3-adamantanol in step b) of the process object of the present invention is from 1 to 3 moles per mole of the salt of formula (VI); 2 moles of 1-amino-3-adamantanol per mole of the salt of formula (VI) are preferably used.

The condensation reaction of step b) is carried out using a suitable condensing agent selected among carbonyldiimidazole, dicyclohexylcarbodiimide, 2,4,6-tri-n-propyl-2,4,6-trioxo-1,3,5,2,4,6-trioxa-triphosphorinane; diciclohexylcarbodiimide is preferably used.

The amount of the condensing agent in step b) is from 1 mole to 1.5 moles per mole of the salt of formula (VI), preferably from 1.1 to 1.3 moles per mole of the salt of formula (VI).

At the end of the reaction of step b) the compound of formula 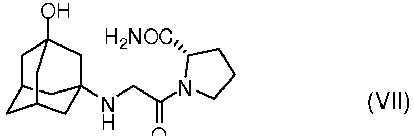 Is obtained.

Step c) of the process object of the present invention provides for the dehydration reaction of compound (VII) to vildagliptin, by treatment with a dehydrating agent in a suitable solvent, optionally in the presence of a base.

The dehydration reaction of step c) is carried out using a suitable dehydrating agent selected among phosphoryl chloride (POCl₃), phosphoric anhydride (P₂O₅), 1,3,5-trichlorotriazine, preferably phosphoryl chloride (POCl₃).

The amount of the dehydrating agent in step c) is from 1 mole to 5 moles per mole of the compound of formula (VII), preferably from 2 to 3 moles per mole of the compound of formula (VII).

The base which is optionally used in step c) is selected among ethylnicotinate, triethylamine, diethylisopropylamine, pyridine, N,N-dimethylaminopyridine, preferably ethylnicotinate.

A preferred practical embodiment of the process object of the present invention is the following.

Chloroacetic acid and L-prolinamide are reacted in methylene chloride to give the salt of formula (VI). The treatment with dicyclohexylcarbodiimide in methylene chloride follows and, without isolation of the resultant product, it is reacted with 1-amino-3-adamantanol to obtain the intermediate of formula (VII). The intermediate is treated with POCl₃ in methylene chloride, optionally in the presence of ethylnicotinate, to obtain vildagliptin.

All the terms used in the present application, unless otherwise indicated, are to be understood in their common meaning as known in the art. Other more specific definitions for certain terms, as indicated in this application, are underlined later and are constantly applied for the whole description and the claims unless a different definition provides specifically a wider meaning.

The term "apolar solvent" refers to a solvent which does not behave as a proton donor. Examples include, without limitations, halogenated hydrocarbons, such as for example, methylene chloride and chloroform, heterocycles, such as for example, tetrahydrofuran and N-methylpyrrolidinone; ethers such as for example, diethyl ether and similar.

Further information about non polar or polar solvents can be found in organic chemistry books or in specialized monographs, for example, Organic Solvents Physical Properties and Methods of Purification, 4th ed., John A. Riddick, et al., Vol II, in "Techniques of Chemistry Series", John Wiley & Sons, NY, 1986. Such solvents are known to the person skilled in the art and it is clear to the person skilled in the art that different solvents and mixtures thereof can be preferred, depending on the specific compounds and on the reaction conditions, being their choice influenced, for example, by solubility and reagent reactivity, by preferred temperature ranges.

Although the present invention has been described in its characterizing features, the equivalents and modifications obvious to the skilled in the art are included in the present invention.

In order to better illustrate the present invention without limiting it, the following examples are now given.

### EXAMPLE 1

28.7 g of L-prolinamide (0.25 mol) and 300 ml of methylene chloride were charged into a reaction flask and the solution was kept under stirring at a temperature of 15°C. 23.63 g of chloroacetic acid (0.25 mol) were added and the reaction mixture was kept under such conditions for one hour. At the end of the reaction the resultant solid was filtered and washed with methylene chloride (2 x 20 ml) and dried in oven under vacuum at 40°C to obtain 49 g of L-prolinamide chloroacetate.
¹H-NMR (DMSO-D6, 300 MHz): δ 4.12 (t, 1 H), 3.90 (s, 2H), 3.15 (t, 2H), 1.84 (m, 2H), 1.82 (m, 2H)
¹³C-NMR (DMSO-D6, 300 MHz): δ 171.33 (C), 169.84 (C), 59.20 (CH), 45.78 (CH₂), 44.73 (CH₂), 30.31 (CH₂), 24.49 (CH₂)

### EXAMPLE 2

28.7 g of L-prolinamide (0.25 mol) and 300 ml of methylene chloride were charged into a reaction flask and the solution was kept under stirring at a temperature of 15°C. 34.73 g of bromoacetic acid were added and the reaction mixture was kept under such conditions for one hour. At the end of the reaction, the resultant solid was filtered and washed with methylene chloride (2 x 20 ml) and dried in oven under vacuum at 40°C to obtain 59.47 g of L-prolinamide bromoacetate.
¹H-NMR (DMSO-D6, 300 MHz): δ 4.12 (t, 1 H), 3.70 (s, 2H), 3.15 (t, 2H), 1.84 (m, 2H), 1.82 (m, 2H)
¹³C-NMR (DMSO-D6, 300 MHz): δ 171.33 (C), 169.84 (C), 59.20 (CH), 45.78 (CH₂), 36.41 (CH₂), 30.31 (CH₂), 24.49 (CH₂)

### EXAMPLE 3

52.15 g of L-prolinamide chloroacetate (0.25 mol) and 300 ml of methylene chloride were charged into a reaction flask. Keeping the temperature at 15°C, 56.74 g of dicyclohexylcarbodiimide (0.28 mol) were gradually added, the temperature was brought to 25°C and the reaction mixture was kept under such conditions for 30 minutes. At the end of the reaction, methylene chloride was removed by distillation under vacuum till obtaining a residue to which 500 ml of tetrahydrofuran were added and keeping the temperature at 25°C, 91.98 g of 3-amino-1-adamantanol (0.55 mol) were added. The temperature was brought to 45°C and reaction mixture was kept under such conditions for about 3 hours. At the end of the reaction, the resultant solid was filtered at warm and the solid was washed with tetrahydrofuran (3 x 70 ml). The mother liquors were distilled up to the removal of about 350 ml of solvent, slowly cooled up to a temperature of 10°C, to obtain a precipitate which was filtered and washed with cold tetrahydrofuran (2 x 50 ml) and dried in oven under vacuum at 50°C to obtain 61.5 g of (2S)-1-[2-[(3-hydroxy-1-adamantyl)amino]-acetyl]pyrrolidin-2-carboamide.
¹H-NMR (DMSO-D6, 300 MHz): δ 4.2 (t, 1 H), 3. 43 (m, 2H), 3.39 (s, 2H), 2.11 (d, 4H), 1.84 (m, 4H), 1.42 (m, 12H).
¹³C-NMR (DMSO-D6, 300 MHz): δ 174.39 (C), 170.56 (C), 68.27 (C), 59.35 (CH), 53.22 (CH₂), 50.70 (CH₂), 45.01 (CH₂), 43.37 (C), 41.67 (CH₂), 40.37 (CH₂), 35.67 (CH₂), 31.00 (C), 30.77 (CH), 29.83 (CH₂), 24.64 (CH₂).

### EXAMPLE 4

6.4 g of (2S)-1-[2-[(3-hydroxy-1-adamantyl)amino]-acetyl]pyrrolidin-2-carboamide (0.0200 mol) and 128 ml of methylene chloride were charged into a reaction flask, the temperature was brought to 10°C and 6.48 ml of phosphorus oxychloride (0.0696 mol) were added. The reaction mixture was brought to the reflux temperature of the solvent and kept under such conditions for four hours. At the end of the reaction, the temperature was brought to 25°C, 20 ml of water were added and the pH was brought to about 9. The organic layer was washed with water (40 ml) and the solvent removed by distillation under vacuum to give a residue which was crystallized with methylethylketone to obtain 4.53 g of vildagliptin.

### EXAMPLE 5

6.4 g of (2S)-1-[2-[(3-hydroxy-1-adamantyl)amino]-acetyl]pyrrolidin-2-carboamide (0.0200 mol), 128 ml of methylene chloride and 1.28 ml of ethylnicotinate (0.0094 mol) were charged into a reaction flask, the temperature was brought to 10°C and 6.48 ml of phosphorus oxychloride (0.0696 mol) were added. The reaction mixture was brought to the reflux temperature of the solvent and kept under such conditions for three hours. At the end of the reaction the temperature was brought to 25°C, 20 ml of water were added and the pH was brought to about 9 (with NaOH 30%). The organic layer was washed with water (40 ml) and the solvent removed by distillation under vacuum to give a residue which was crystallized with methylethylketone to obtain 3.63 g of vildagliptin.

### EXAMPLE 6

6.4 of (2S)-1-[2-[(3-hydroxy-1-adamantyl)amino]-acetyl]pyrrolidin-2-carboamide (0.0200 mol) and 60 ml of dimethylformamide were charged into a reaction flask and the temperature was brought to 0°C. 2.39 g of trichlorotriazine (0.013 mol) were added and the reaction mixture was kept under such conditions for one hour. The temperature was then brought to 25°C and the reaction mixture was kept under such conditions for four hours. At the end of the reaction, 30 ml of an aqueous solution of sodium chloride at 15% and 120 ml of methylene chloride were added and the pH was brought to about 9 (with NaOH 30%). The solvent was removed by distillation under vacuum to give a residue which was crystallized with methylethylketone to obtain 3.21 g of vildagliptin.

## Claims

1. A process for the synthesis of vildagliptin comprising:
a) the salification of L-prolinamide with haloacetic acid (XCH₂COOH) in a suitable solvent to obtain the salt of formula (VI) 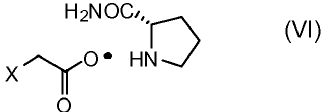 wherein X is bromine or chlorine
b) the condensation of the salt of formula (VI) by reaction with a condensing agent and subsequent reaction with 1-amino-3-adamantanol in a suitable solvent to give the intermediate of formula (VII), without the isolation of the condensation product 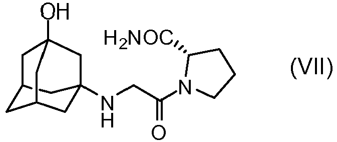
c) the dehydration of the compound (VII) to vildagliptin, by treatment with a dehydrating agent in a suitable solvent, optionally in the presence of a base.

2. A process according to claim 1 wherein in steps a), b) and c) the solvents, the same or different for each step, are apolar solvents selected among tetrahydrofuran, methylene chloride and 2-methyltetrahydrofuran.

3. A process according to claim 2 wherein the solvent is tetrahydrofuran or methylene chloride.

4. A process according to claim 1 wherein in step b) the condensing agent is selected among carbonyldiimidazole, dicyclohexylcarbodiimide and 2,4,6-tri-n-propyl-2,4,6-trioxo-1,3,5,2,4,6-trioxa-triphosphorinane.

5. A process according to claim 4 wherein the condensing agent is dicyclohexylcarbodiimide.

6. A process according to claim 1 wherein in step c) the dehydrating agent is selected among phosphoryl chloride (POCl₃), phosphoric anhydride (P₂O₅) and 1,3,5-trichlorotriazine.

7. A process according to claim 6 wherein the dehydrating agent is phosphoryl chloride (POCl₃).

8. A process according to claim 1 wherein in step c) the base is selected among ethylnicotinate, triethylamine, diethylisopropylamine, pyridine and N,N-dimethylaminopyridine.

9. A process according to claim 8 wherein the base is ethylnicotinate.

10. An intermediate for the synthesis of vildagliptin of formula (VI) 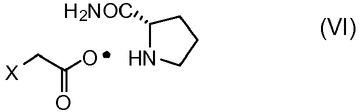 wherein X is bromine or chlorine.

11. Use of the compound of formula (VII) for the synthesis of vildagliptin.

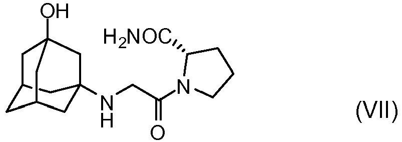

## Patentansprüche

1. Verfahren zur Synthese von Vildagliptin, umfassend:
a) die Salzbildung von L-Prolinamid mit Halogenessigsäure (XCH₂COOH) in einem geeigneten Lösungsmittel zur Gewinnung des Salzes der Formel (VI) 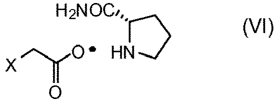 worin X Brom oder Chlor bedeutet.
b) die Kondensation des Salzes der Formel (VI) durch Reaktion mit einem Kondensationsmittel und anschließende Reaktion mit 1-Amino-3-adamantanol in einem geeigneten Lösungsmittel zur Gewinnung des Zwischenprodukts der Formel (VII), ohne die Isolierung des Kondensationsprodukts 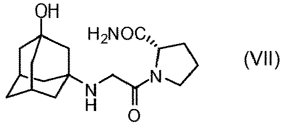
c) die Entwässerung der Verbindung (VII) zu Vildagliptin durch Behandlung mit einem entwässernden Mittel in einem geeigneten Lösungsmittel, gegebenenfalls in Gegenwart einer Base.

2. Verfahren nach Anspruch 1, wobei in Schritten a), b) und c) die Lösungsmittel, gleich oder verschieden für jeden Schritt, unpolare Lösungsmittel sind, ausgewählt unter Tetrahydrofuran, Methylenchlorid und 2-Methyltetrahydrofuran.

3. Verfahren nach Anspruch 2, wobei das Lösungsmittel Tetrahydrofuran oder Methylenchlorid ist.

4. Verfahren nach Anspruch 1, wobei in Schritt b) das Kondensationsmittel ausgewählt ist unter Carbonyldiimidazol, Dicyclohexylcarbodiimid und 2,4,6-Tri-n-propyl-2,4,6-trioxo-1,3,5,2,4,6-trioxa-triphosphorinan.

5. Verfahren nach Anspruch 4, wobei das Kondensationsmittel Dicyclohexylcarbodiimid ist.

6. Verfahren nach Anspruch 1, wobei in Schritt c) das entwässernde Mittel ausgewählt ist unter Phosphorylchlorid (POCl₃), Phosphorsäureanhydrid (P₂O₅) und 1,3,5-Trichlortriazin.

7. Verfahren nach Anspruch 6, wobei das entwässernde Mittel Phosphorylchlorid (POCl₃) ist.

8. Verfahren nach Anspruch 1, wobei in Schritt c) die Base ausgewählt ist unter Ethylnicotinat, Triethylamin, Diethylisopropylamin, Pyridin und N,N-Dimethylaminopyridin.

9. Verfahren nach Anspruch 8, wobei die Base Ethylnicotinat ist.

10. Zwischenprodukt der Formel (VI) 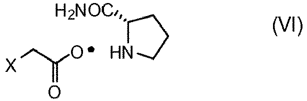 worin X Brom oder Chlor ist, zur Synthese von Vildagliptin.

11. Verwendung der Verbindung der Formel (VII) zur Synthese von Vildagliptin.

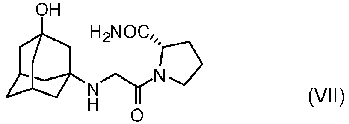

## Revendications

1. Procédé de synthèse de vildagliptine comprenant :
a) la salification du L-prolinamide avec de l'acide haloacétique (XCH₂COOH) dans un solvant approprié pour obtenir le sel de formule (VI) 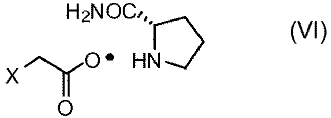 dans lequel X est du brome ou du chlore
b) la condensation du sel de formule (VI) par réaction avec un agent de condensation et la réaction subséquente avec du 1-amino-3-adamantanol dans un solvant approprié pour donner l'intermédiaire de formule (VII), sans isoler le produit de condensation 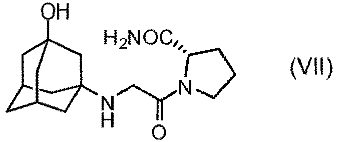
c) la déshydratation du composé (VII) en vildagliptine, par traitement avec un agent déshydratant dans un solvant approprié, facultativement en présence d'une base.

2. Procédé selon la revendication 1 dans lequel dans les étapes a), b) et c) les solvants, identiques ou différents pour chaque étape, sont des solvants apolaires sélectionnées parmi le tétrahydrofurane, le chlorure de méthylène et le 2-méthyltétrahydrofurane.

3. Procédé selon la revendication 2 dans lequel le solvant est le tétrahydrofurane ou le chlorure de méthylène.

4. Procédé selon la revendication 1 dans lequel dans l'étape b) l'agent de condensation est sélectionné parmi le carbonyldiimidazole, le dicyclohexylcarbodiimide et le 2,4,6-tri-n-propyl-2,4,6-trioxo-1,3,5,2,4,6-trioxa-triphosphorinane.

5. Procédé selon la revendication 4 dans lequel l'agent de condensation est le dicyclohexylcarbodiimide.

6. Procédé selon la revendication 1 dans lequel dans l'étape c) l'agent déshydratant est sélectionné parmi le chlorure de phosphoryle (POCl₃), l'anhydride phosphorique (P₂O₅) et la 1,3,5-trichlorotriazine.

7. Procédé selon la revendication 6 dans lequel l'agent déshydratant est le chlorure de phosphoryle (POCl₃).

8. Procédé selon la revendication 1 dans lequel dans l'étape c) la base est sélectionnée parmi le nicotinate d'éthyle, la triéthylamine, la diéthylisopropylamine, une pyridine et la N,N-diméthylaminopyridine.

9. Procédé selon la revendication 8 dans lequel la base est le nicotinate d'éthyle.

10. Intermédiaire pour la synthèse de vildagliptine de formule (VI) 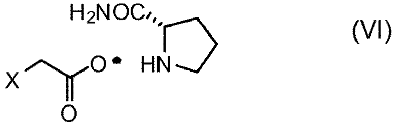 dans lequel X est du brome ou du chlore

11. Utilisation du composé de formule (VII) pour la synthèse de vildagliptine.
